# EUROPEAN PATENT APPLICATION

(11) **EP 4 491 722 A1**
(43) Date of publication of application: **15.01.2025**
(21) Application number: 24181643.8
(22) Date of filing: 12.06.2024
(51) Int. Cl.: C12N 9/64, A23C 19/00, C12P 21/02

(54) **METHOD FOR THE PRODUCTION OF DRIED RENNET**

(30) Priority: 30.06.2023 IT 202300013602
(71) Applicant: Calza Clemente S.r.l., 26020 Acquanegra Cremonese CR (IT)
(72) Inventor: CALZA, Roberto, 26020 CREMONA (IT)
(74) Representative: Modiano, Micaela Nadia

(57) **Abstract**

A method (1) for producing dried rennet (2) using bovine abomasa (3), which comprises the steps of: (I) grinding the abomasa (3); (II) adding a salt to the ground mass (4) of abomasa (3) in order to limit bacterial proliferation; (III) drying the ground mass (5) with added salt in a static dryer (6) at a temperature comprised between 15 °C and 50 °C in order to extract moisture from the mass (5), which has initially a water content comprised between 50% and 90%, until a dried ground mass (7) having a water content comprised between 1% and 10%, preferably between 3% and 7%, is obtained; (IV) finely grinding the ground and dried mass (7), obtaining a dried rennet (2) that is directly usable in the dairy industry.

## Description

The present invention relates to a method for the production of dried rennet.

In some specific territories, which include some French and Swiss Alpine regions, the use of dried calf rennet as a coagulant for the production of some typical cheeses is known. For example, the cheeses known as Comtè and Beaufort, which provide for the exclusive adoption of this specific coagulant during their production, can be mentioned.

The production of dried calf rennet, according to the prior art, involves inflating an abomasum of a suckling calf with air and then drying it in ventilated rooms with controlled humidity and temperature. Once dried, said abomasa will have to be stripped of their moist parts (in particular, the bottom of each individual piece, at which, by gravity, the liquid residues will accumulate, percolating), also eliminating all the undesirable parts (among which it is possible to mention filaments of fat, regions marked by possible stains, and also parts that have not properly dried).

The cheesemaker then divides the product thus dried into small pieces, then he will subject the small pieces to maceration in an acid whey for several hours, keeping everything at a temperature of about 45°C, in order to extract the enzyme that will be later used in cheesemaking.

The prior art technique for the production of dried rennet entails that a certain amount of raw material must necessarily be discarded: the drying of the inflated abomasum causes the accumulation of a moist fraction in its lower region, which must be discarded, since it therefore does not reach the necessary degree of drying. Likewise, some additional parts of the abomasum might not reach the necessary level of drying: these also will have to be discarded.

The production yield expected in the prior art, therefore, is rather low.

Moreover, all these operations necessary to select and eliminate the parts to be discarded require the intervention of specialized personnel and therefore of labor which heavily affects the final cost of dried rennet.

It should be specified that the drying step provided in the prior art leads to the growth of a very complex bacterial flora, which is also linked to the occurrence of putrefactive phenomena. Reduction of this high bacterial load occurs during the whey extraction step. However, the amount of gas-producing bacteria is, in many cases, still excessively high, which results in the generation of defects in the cheeses that will be produced using such rennet.

Moreover, this drying step is particularly energy-intensive, since it is necessary to dry abomasa which are inflated with air (and therefore very bulky), leading to the need to have a large drying room (with controlled temperature and humidity).

Finally, it should be pointed out that all these potential defects of dried rennet and the subjectivity through which the individual operator selects the parts to be discarded and the duration of individual treatments lead to a very high qualitative variability of the rennet, which can also be attributed to the chemical-physical variability of the individual abomasum. Therefore, it does not seem possible to have a homogeneous raw material that can be handled uniformly by the cheesemaker for cheese production.

The aim of the present invention is to solve the above problems by providing a method for the production of dried rennet that allows to minimize raw material waste.

Within this aim, an object of the invention is to provide a method for the production of dried rennet having a high production yield.

Another object of the invention is to provide a method for the production of dried rennet that requires very little labor, thereby also reducing the costs associated with it and the subjectivity of each individual operator's interventions.

Another object of the invention is to provide a method for the production of dried rennet that has a lower energy consumption than that required by the background art.

Another object of the invention is to provide a method for the production of dried rennet having a lower bacterial load than that provided in the known background art, thus ensuring a higher yield in cheese production.

Another object of the invention is to provide a method for the production of dried rennet that is very homogeneous and therefore easier to be used owing to the standardization of its characteristics.

A further object of the present invention is to provide a method for the production of dried rennet that has low costs and is relatively simple to provide in practice and of assured application.

This aim and these and other objects that will become more apparent hereinafter are achieved by a method for producing dried rennet using bovine abomasa, characterized in that it comprises the steps of:
I. Grinding the abomasa;
II. Adding a salt to the ground mass of abomasa in order to limit bacterial proliferation, said salt being introduced with a percentage by mass comprised between 0.2% and 10%;
III. Drying the ground mass with added salt in a static dryer at a temperature comprised between 15 °C and 50 °C, preferably between 20 °C and 35 °C, performing a closed cycle of air at controlled temperature and humidity in order to extract moisture from the ground mass with added salt, which has initially a water content comprised between 50% and 90%, preferably between 60% and 80%, until a dried ground mass having a water content comprised between 1% and 10%, preferably between 3% and 7%, is obtained;
IV. Finely grinding said ground and dried mass, obtaining a dried rennet that is directly usable in the dairy industry.

Further characteristics and advantages of the invention will become better apparent from the description of a preferred but not exclusive embodiment of the method for producing dried rennet, illustrated by way of non-limiting example in the accompanying drawings, wherein:

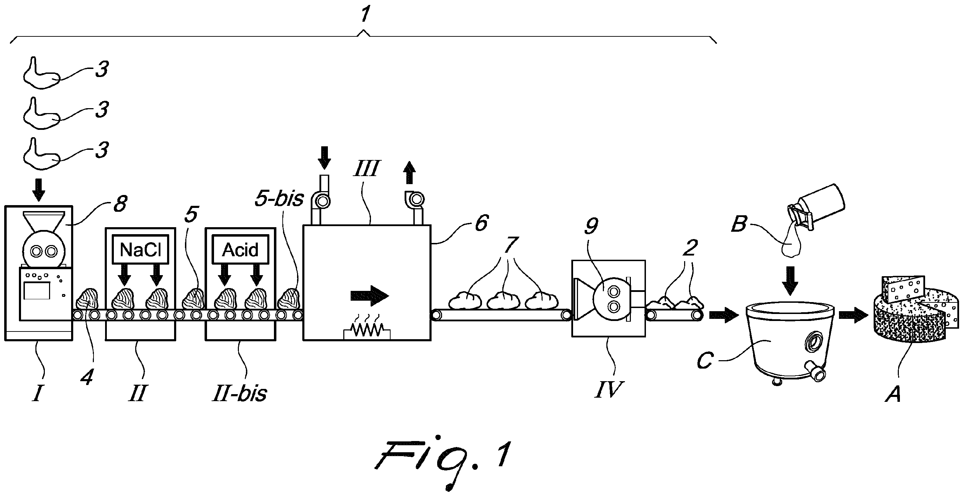

Figure 1 is a schematic view of a possible flow chart of the method for producing dried rennet according to the invention.

With reference to the figure, a method for the production of dried rennet 2 is generally designated by the reference numeral 1.

The graphical representation of the components adopted in Figure 1 is only intended to make the interpretation of the method 1 intuitive: it is not excluded that different components may be used which allow to achieve the same aim and objects using distinct techniques.

The method 1 according to the invention provides for the use of bovine abomasa 3.

The abomasum is one of the four compartments into which the stomach of ruminants is divided, more particularly the one which constitutes the stomach proper.

The method 1 according to the invention consists in performing a series of consecutive steps.

During a first step (I) it is necessary to grind the abomasa: grinding is preferably be carried out by providing a ground mass 4 that is as fine as possible (to the extent allowed by the equipment in use in the food industry), since this will enable the ground mass 4 to respond best to the subsequent steps in the process.

During a second step (II) it is necessary to add a salt to the ground mass 4 of abomasa in order to limit bacterial proliferation: the salt can be advantageously introduced with a percentage by mass comprised between 0.2% and 10%. A ground mass 5 of abomasa with added salt is obtained at the end of said step (II).

A subsequent step (III) provides for drying in a static dryer 6 at a temperature comprised between 15 °C and 50 °C, preferably between 20 °C and 35 °C, by performing a closed cycle of air at controlled temperature and humidity, in order to extract moisture from the ground mass 5 with added salt.

The ground mass 5 with added salt arrives at the dryer 6 with an initial water content comprised between 50% and 90%, preferably between 60% and 80%; at the end of the drying step (III), a dried ground mass 7 is obtained which has a water content comprised between 1% and 10%, preferably between 3% and 7%.

During a subsequent step (IV) of the method 1 according to the invention, the ground and dried mass 7 is finely ground, resulting in a dried rennet 2 directly usable in the dairy industry.

It should be noted that the method 1 according to the invention, in contrast to all the techniques provided in the prior art, provides that the abomasa 3 used in step (I) can be frozen and are, therefore, at a temperature comprised between 0 °C and -100 °C, preferably between -10 °C and -50 °C, even more preferably between -15 °C and -20 °C.

In fact, the preservation and storage technique used in the background art involves freezing the abomasa 3, since this allows to stop bacterial growth and halt decomposition.

The prior art instead provides that the abomasa 3 are thawed and then inflated before being subjected to drying at room temperature (i.e., by way of indication, between 15 °C and 30 °C): all these operations result in considerable bacterial proliferation, which can lead to problems of various kinds, compromising the quality of the dried rennet that it produces and consequently increasing the defects in the cheeses A provided by using it.

Performing the grinding step (I) directly on the frozen abomasa 3 is a significant advantage, because it allows to keep the bacterial load under control (minimizing it) during this step (which will result in a minimization thereof on the finished product (dried rennet 2).

Since the abomasa 3 are generally stored in cold rooms in which a temperature comprised between -15 °C and -20 °C (frequently around -18 °C) is maintained, it therefore turns out to be possible to extract the abomasa 3 from the room and feed them directly into the shredding apparatus 8 through which the grinding step (I) is performed.

With reference to a method of unquestionable interest in practice and in application, the salt introduced in step (II) may advantageously be sodium chloride.

Sodium chloride, or table salt, by removing the water that would cause microorganisms to proliferate, regulates meat fermentation processes, containing bacterial growth.

Sodium chloride must be introduced into the mass 4 of ground abomasa 3, with a percentage by mass comprised between 1% and 5%, preferably between 1.5% and 3.5%, even more preferably between 2% and 3%.

In general, the salt introduced in step (II) may be distributed on the outer surface of mass 4 or, through suitable mixers, incorporated into said mass 4.

In any case, a mass 5 with added salt is obtained at the end of step (II).

Moreover, it is specified that the method 1 according to the invention may advantageously comprise an optional step (II-bis) in which the acidity of the ground mass with added salt is adjusted if it has a pH value outside the range 2.00-4.00.

Obviously, this step (II-bis) is unnecessary if the mass 5 (ground abomasa 3 with added salt) already has a pH value comprised between 2.00 and 4.00 (i.e., already has the correct degree of acidity); instead, it is necessary to perform said step whenever the acidity of the mass 5 does not fall within the identified range (2 ≤ pH ≤ 4).

Adjustment of the acidity of the mass 5, for the purpose of giving a pH value comprised between 2.00 and 4.00, may be carried out through the addition of an acid of a type chosen from hydrochloric acid, citric acid, lactic acid, ascorbic acid, tartaric acid, phosphoric acid, orthophosphoric acid, boric acid, malic acid, fumaric acid, metartaric acid, adipic acid, succinic acid, and the like. Such additional step (II-bis) must be performed after step (II) and before step (III).

With reference to a method of assured implementation, the acid is chosen in particular between citric acid and hydrochloric acid.

Moreover, it is specified that the drying step (III) involves subjecting to higher temperatures the ground mass 5 with added salt, possibly subjected to pH adjustment through the addition of an acid (this being the mass 5-bis in this case), if the latter has a high initial water content, even close to the upper limits of the said ranges.

In practice, if it is found that the moisture content of the mass 5 (or mass 5-bis) is particularly high (close to the upper limits previously indicated around 80% by mass or possibly even higher than that), a drying step (III) is carried out and is performed at higher temperatures (for example, close to the upper limit identified earlier with the value of 50 °C or, according to the preferred solution, with the value 35 °C).

If the moisture is instead lower (close to the lower limits previously indicated in the vicinity of 60% by mass or possibly even lower than the same), a drying step (III) is performed at lower temperatures (for example, close to the upper limit identified earlier with the value 15 °C or, according to the preferred solution, with the value 20 °C).

Moreover, the step (III) of drying the ground mass 5 with added salt, possibly subjected to pH adjustment through the addition of an acid (this being the mass 5-bis in this case), may favorably have a longer duration. In particular, when said mass 5 (or mass 5-bis) has a high initial water content, even close to the upper limits of the cited ranges (on the order of 80% by mass), the duration of the drying step (II) will be longer than when the mass 5 (or mass 5-bis) has a low initial water content (for example, on the order of 60% by mass).

With regard to the step (IV) of fine grinding of the ground and dried mass 7, it is specified that it intended to generate dried rennet flakes 2 ready for use in the dairy industry. The flake shape ensures easy dosage and use in dairies. The grinding of step (IV) is performed by means of a food-grade grinding device 9 of a known type (which allows to obtain powders of even very fine grain size).

The dried rennet 2 is extremely stable. For this reason, it can be stored in non-vacuum food containers (although resorting to this option or to controlled atmosphere packaging is not excluded), which will be stored at a controlled temperature comprised between 0 °C and 15 °C (preferably between 2 °C and 8 °C). In these storage conditions (thus packaged and stored) the dried rennet 2 is found to be stable for a very long time (potentially even several years, providing a preferred shelf life of about 24 months).

It is specified that the abomasa 3 used in method 1 according to the invention are preferably abomasa 3 from suckling calves.

The use in the dairy industry of the dried rennet 2 (previously macerated in whey at a predefined temperature, for example comprised between 35 °C and 50 °C depending on the specific requirements of the cheese A to be produced) involves the addition thereof (according to predefined dosages) to milk B within C containers within which curd is formed (also by virtue of specific temperature conditions).

The present invention also extends its protection to a dried rennet 2 obtained from bovine abomasa 3, which is constituted by powdery flakes that homogeneously and uniformly comprise a water content comprised between 1% and 10%, preferably between 3% and 7%, and have a pH value between 2.00 and 4.00.

In greater detail, the dried rennet 2 according to the invention is obtained via a method 1 of the type described above.

Advantageously, the present invention solves the problems set forth above by providing a method 1 for the production of dried rennet 2 that allows minimizing raw material waste.

In fact, the grinding step (I) provides for including the frozen abomasa 3 in their entirety: once they have been ground, a homogeneous mass 4 is produced, which (later in the method 1) is dried in its entirety.

Thus, there are no parts of the abomasa 3 that are subject to stagnation of percolated liquids as is the case in the background art in which such parts must be discarded.

Conveniently, the method 1 according to the invention has a high production yield, precisely because no parts of the abomasa 3 are discarded. In the background art, it is left to the subjective assessment of the operator to estimate the amount of material to be discarded, resulting in a large difference in yield also as a function of such subjective assessments. What is more, small defects of the abomasa 3 in the present invention are ignored (unlike in the background art, where they are subject to rejection) because the ground mass 4 is constituted by the ground mixture of a plurality of different abomasa 3 and therefore any defects are strongly diluted in the total mass, becoming irrelevant (they do not determine any negative effect on the dried rennet 2 being provided, let alone on the cheese A to be produced using the same).

Favorably, the method 1 according to the invention requires very little labor (and potentially can even be fully automated), thus also reducing the costs associated with said labor and the subjectivity of each individual operator's interventions.

Advantageously, the method 1 according to the invention has lower energy consumption than in the background art: this is due, in particular, to the fact that the static dryer 6 can be significantly smaller in size than is required to process the same amount of raw material according to the background art (of course, providing climate control for a smaller room results in lower costs).

In particular, it is specified that according to the background art, the average energy consumption for processing a single abomasum 3 corresponds to about 2.4 kWh.

If one adopts the process 1 according to the invention, the estimated energy consumption for processing each abomasum 3 is about 0.70 kWh, resulting in a reduction in energy consumption of about 70%.

Positively, the method 1 according to the invention makes it possible to produce a dried rennet 2 having a lower bacterial load than envisaged in the background art, ensuring a higher yield in the production of cheeses A.

In fact, the drying step provided in the background art generates a very complex bacterial flora (and/or fauna), also as a result of putrefactive processes, which is subsequently "depressed" during the whey extraction step. However, the amount of gas-producing bacteria present is often too high, and this generates a rather high percentage of defective cheeses. The method 1 according to the invention makes it possible to control the bacterial load by virtue of the possibility of adjusting the chemical parameters of the ground mass 4 (an operation that is not possible with whole abomasa 3); steps (II) and (II-bis) are in fact provided for this purpose and bacterial proliferation is inhibited with them, thanks to the introduction of salt (preferably food-grade sodium chloride) and the ideal degree of acidity is maintained (maintaining conditions that do not favor bacterial proliferation). Such operations could in no way be performed in the background art.

Usefully, the method 1 according to the invention allows to produce a dried rennet 2 that is very homogeneous and therefore easier to use by virtue of the standardization of its characteristics.

The traditional process according to the background art undergoes qualitative variability due to the chemical/physical variability of each individual abomasum 3.

By adopting the method 1 according to the invention, which involves grinding and mixing a considerable number of abomasa 3, it is possible to create masses 4 (to which salt is added later, becoming masses 5, which in turn are optionally adjusted in their acidity by the addition of an acid, becoming masses 5-bis) that are chemically/physically homogeneous and also allow to perform analytical checks which are representative of the batch. This would be totally impossible when operating with whole abomasa 3.

Validly, the method 1 for the production of dried rennet 2 and the dried rennet 2 according to the invention are relatively simple to provide in practice and cost-effective: such characteristics make the method 1 and the dried rennet 2 according to the invention innovations of assured application.

The invention thus conceived is susceptible of numerous modifications and variations, all of which are within the scope of the appended claims; all the details may furthermore be replaced with other technically equivalent elements.

In the embodiments shown, individual characteristics, given in relation to specific examples, may actually be interchanged with other different characteristics that exist in other embodiments.

In practice, the materials used, as well as the dimensions, may be any according to the requirements and the state of the art.

The disclosures in Italian Patent Application No. 102023000013602 from which this application claims priority are incorporated herein by reference.

Where technical features mentioned in any claim are followed by reference signs, such reference signs have been inserted for the sole purpose of increasing the intelligibility of the claims and accordingly such reference signs do not have any limiting effect on the interpretation of each element identified by way of example by such reference signs.

## Claims

1. A method for producing dried rennet (2) using bovine abomasa (3), **characterized in that** it comprises the steps of:
I) grinding the abomasa (3);
II) adding a salt to the ground mass (4) of abomasa (3) in order to limit bacterial proliferation, said salt being introduced with a percentage by mass comprised between 0.2% and 10%;
III) drying the ground mass (5) with added salt in a static dryer (6) at a temperature comprised between 15 °C and 50 °C, preferably between 20 °C and 35 °C, performing a closed cycle of air at controlled temperature and humidity in order to extract moisture from the ground mass (5) with added salt, which has initially a water content comprised between 50% and 90%, preferably between 60% and 80%, until a dried ground mass (7) having a water content comprised between 1% and 10%, preferably between 3% and 7%, is obtained;
IV) finely grinding said ground and dried mass (7), obtaining a dried rennet (2) that is directly usable in the dairy industry.

2. The method according to claim 1, **characterized in that** said abomasa (3) used in said step (I) are frozen and are at a temperature comprised between 0 °C and -100 °C, preferably between -10 °C and -50 °C, even more preferably between -15 °C and -20 °C, said freezing being provided for their preservation during storage.

3. The method according to one or more of the preceding claims, **characterized in that** said salt is sodium chloride, introduced in the mass (4) of ground abomasa (3) with a percentage by mass comprised between 1% and 5%, preferably between 1.5% and 3.5%, even more preferably between 2% and 3%.

4. The method according to one or more of the preceding claims, **characterized in that** it comprises an optional step (II-bis) of adjusting the acidity of the ground mass (5) with added salt if it has a pH value outside the range of 2.00 to 4.00, in order to give a pH value comprised between 2.00 and 4.00 through the addition of an acid of a type chosen from hydrochloric acid, citric acid, lactic acid, ascorbic acid, tartaric acid, phosphoric acid, orthophosphoric acid, boric acid, malic acid, fumaric acid, metartaric acid, adipic acid, succinic acid, and the like, said additional step (II-bis) being performed after said step (II) and before said step (III).

5. The method according to the preceding claim, **characterized in that** said acid is chosen between citric acid and hydrochloric acid.

6. The method according to one or more of the preceding claims, **characterized in that** said drying step (III) provides for subjecting to higher temperatures, within the quoted ranges, said ground mass (5, 5-bis) with added salt, optionally subjected to pH adjustment by means of the addition of an acid, when said mass (5, 5-bis) has a high initial water content, even close to the upper limits of the cited ranges.

7. The method according to one or more of the preceding claims, **characterized in that** said step (III) of drying said ground mass (5, 5-bis) with added salt, optionally subjected to pH adjustment by adding an acid, has a longer duration, when said mass (5, 5-bis) has a high initial water content, even close to the upper limits of the cited ranges.

8. The method according to one or more of the preceding claims, **characterized in that** said step (IV) of fine grinding of said ground and dried mass (7) is intended to generate flakes of dried rennet (2) ready for use in the dairy industry.

9. The method according to one or more of the preceding claims, **characterized in that** said abomasa (3) are abomasa (3) of suckling calves.

10. Dried rennet of the type obtained from bovine abomasa, **characterized in that** it is constituted by powdery flakes which homogeneously and uniformly comprise a water content comprised between 1% and 10%, preferably between 3% and 7%, and have a pH value comprised between 2.00 and 4.00.

11. Dried rennet according to claim 10, **characterized in that** it is obtained by means of a method (1) according to at least one of claims 1 to 9.
